# EUROPÄISCHE PATENTSCHRIFT

(11) **EP 0 630 618 B1**
(45) Veröffentlichungstag und Bekanntmachung des Hinweises auf die Patenterteilung: **02.12.1998**
(21) Anmeldenummer: 94108239.8
(22) Anmeldetag: 27.05.1994
(51) Int. Cl.: A61B 17/34, A61M 25/04

(54) **Applikationsbesteck für Katheter o.dgl.**
Surgical appliance for placing catheters or the like
Instrument chirurgical pour cathéters ou choses semblables

(30) Priorität: 28.05.1993 DE 4317754
(43) Veröffentlichungstag der Anmeldung: 28.12.1994
(73) Patentinhaber: Krütten, Viktor, D-65510 Idstein (DE)
(72) Erfinder: Krütten, Viktor, D-65510 Idstein (DE)
(74) Vertreter: Müller, Eckhard, Dr.

(56) Entgegenhaltungen:
- EP-A- 0 475 324
- DE-A- 3 422 597
- DE-A- 3 713 828
- US-A- 4 716 901
- US-A- 5 112 310

## Beschreibung

Die Erfindung bezieht sich auf ein Applikationsbesteck nach dem Oberbegriff des Anspruchs 1.

Ein solches Applikationsbesteck ist bekannt aus der US-A-4 716 901.

Derartige Sonden oder Katheter dienen zum Entleeren des Inhaltes von Körperorganen und -wegen oder zum Einbringen von Substanzen in den Körper, wie bspw. zur Einführung von Nahrung in den Magen. Zum Plazieren der Sonden oder Katheter werden Applikationsbestecke benutzt.

So wird bspw. bei der perkutanen endoskopischen Gastrostomie zunächst eine Punktionskanüle in den Magen eingeführt und anschließend die Punktionsnadel entfernt. Danach wird der Führungsfaden gelegt, wobei eine Einführhilfe für den Faden auf die Kunststoffkanüle gesteckt und danach der Faden in den Magen eingeführt wird. Anschließend wird die Einführhilfe wieder entfernt und der Faden im Magen mit einer Biopsiezange erfaßt und herausgezogen. Die Sonde wird dann mit ihrer Fixierschlaufe am Führungsfaden befestigt, um durch langsamen Zug am distalen Fadenende die Sonde zu plazieren. Schließlich wird die Sonde zusammen mit der Kunststoffkanüle durch die Bauchwand nach außen gezogen, bis die elastische Rückhaltescheibe der Sonde an der Mageninnenwand anliegt. Ein wesentlicher Nachteil dieser Fadendurchzugs-Methode ist, daß zur Kontrolle der Punktionsstelle und zum Legen des Führungsfadens ein Endoskop erforderlich ist. Darüber hinaus ist die Anwendung der perkutanen endoskopischen Gastrostomie in vielen Fällen nicht anwendbar, wie z. B. bei Stenosen in der Speiseröhre, so daß es nicht mehr möglich ist, ein Gastroskop und auch die am Katheter bzw. der Sonde befindliche Halteplatte durch die Speiseröhre einzuführen. Darüber hinaus können sich bei der Benutzung von Gastroskopie-Kathetern oberhalb des Magens weitere Stenosen bilden, so daß der Katheter bzw. die Sonde möglicherweise noch entfernt, aber kein neuer Katheter mehr gesetzt werden kann.

Auch ist es schon bekannt, die Sonde nicht über den Schlund, sondern direkt über die Bauchdecke einzuführen. Bei diesem sogenannten Direktpunktionsverfahren dient als Rückhalteelement ein mit Luft gefüllter Latex- oder Silikonballon. Gegenüber dem vorgenannten Verfahren mit einer eine Rückhalteplatte aufweisenden Sonde ist diese Punktionsmethode jedoch mit dem Nachteil behaftet, daß wegen der Ballonform des Rückhalteelementes ein Abdichten der Sonde gegenüber der Mageninnenwandung nur bedingt möglich ist. Zudem neigen derartige Ballons unter dem Einfluß der Magensäure dazu, daß sie undicht werden oder die im Ballon befindliche Luft entweicht und dadurch der Rückhalteeffekt bzw. die Fixierung zwischen Magenvorderwand und Bauchwand aufgehoben ist.

Aus der US-A-5 112 310 ist ein Applikationsbesteck in kombination mit einem Katheter bekannt, wobei das Applikationsbesteck eine Kanüle und eine darin geführte Nadel aufweist und der Katheter eine Rückhalteeinrichtung in Form eines Ballons oder eines "Korbes" aufweist.

Davon ausgehend liegt der Erfindung die Aufgabe zugrunde, ein Applikationsbesteck der eingangs genannte Art dahingehend weiterzubilden, daß das Plazieren der Sonde bzw. des Katheters wesentlich vereinfacht ist und auch bei Stenosen in der Speiseröhre oder oberhalb des Magens noch möglich ist.

Diese Aufgabe wird durch die kennzeichnenden Merkmale des Anspruchs 1 gelöst.

Durch die Erfindung ist erreicht, daß bspw. die Sonde oder der Katheter nicht mehr über den Schlund, sondern direkt in den Körper zu dem jeweiligen Organ oder Körperhöhle eingebracht werden kann, wobei aber durch die Rückhalteplatte ein sicheres Abdichten der Sonde an der jeweiligen Körperinnenwandung erreicht ist und ohne daß es einer endoskopischen Kontrolle bedarf. Dabei wird die Punktionskanüle mit eingesteckter Punktionsnadel bspw. in den Magen eingeführt und anschließend die Punktionsnadel entfernt. Danach wird die Sonde oder der Katheter mittels einer Schiebehilfe mit der Rückhalteplatte voran in die Punktionskanüle eingeführt. Beim Einschieben legt sich die aus Kunststoff, insbesondere aus Silikon, bestehende Rückhalteplatte entgegengesetzt der Schieberichtung nach hinten um, wobei die Durchführung der Punktionskanüle radial aufgeweitet wird und die Sonde mit Rückhalteplatte in den Magen eindringt. Danach wird die Schiebehilfe entfernt und die Sonde mit der Punktionskanüle durch die Bauchwand langsam nach außen gezogen, bis die Rückhalteplatte an der Magenvorderwand anliegt.

Nach einer ersten besonderen Ausführungsform der Erfindung ist es vorgesehen, daß die Punktionskanüle im Bereich ihres Schaftes wenigstens eine, vorzugsweise vier Längsschlitze mit am vorderen Ende der Punktionskanüle angeordneten Sollbruchstellen aufweist, welche beim Durchschieben der Schiebehilfe mit Rückhalteplatte unter Aufspreizen der durch die Längsschlitze gebildeten segmentförmigen Zungen aufbrechen. Da die Zungen der Punktionskanüle in ihrem vorderen Bereich zunächst noch zusammengehalten sind, läßt sich die Funktionskanüle mit Punktionsnadel in einfacher Weise in den Körper einführen, ohne daß das Gewebe außerhalb der Punktionsstelle in Mitleidenschaft gezogen wird.

Alternativ hierzu ist es nach der Erfindung auch vorgesehen, daß die Punktionskanüle im Bereich ihres Schaftes wenigstens eine, vorzugsweise vier durchgehende Längsschlitze aufweist, wobei die durch die Längsschlitze gebildeten segmentförmigen Zungen mittels umfangsseitig der Punktiernadel gebildeten Führungen zusammengehalten und bei Einführen der Schiebehilfe mit der Rückhalteplatte aufspeizbar sind. In eingeschobener Stellung der Punktionsnadel sind die Zungen der Punktionskanüle dicht an der Punktionsnadel gehalten, so daß das Einführen der Punktionskanüle mit eingesteckter Punktionsnadel nochmals verbessert ist.

Die Führungen an der Punktionsnadel können als nach radial außen vorstehende, in Längsrichtung verlaufende Führungsstege ausgebildet sein, welche sich mit den dazwischen zu liegen kommenden Zungen zu einer i. w. geschlossene zylindrischen Außenmantelfläche ergänzen. Insoweit können die Führungsstege i. w. T-förmig ausgebildet sein, in welche die Zungen mit etwa stufenförmig abgesetzen Seitenrändern eingreifen. Hierdurch ist die Verletzungsgefahr und auch der Widerstand beim Einschieben der Punktionskanüle mit aufgesteckter Punktionsnadel wesentlich reduziert.

Die Handhabbarkeit des erfindungsgemäßen Applikationsbestecks kann nach einer Ausgestaltung der Erfindung noch dadurch verbessert werden, daß die Punktionskanüle an ihrem hinteren Ende einen Einführtrichter aufweist, wodurch das Einführen der Injektionskanüle aber auch der Schiebehilfe wesentlich erleichtert ist.

Vorteilhafterweise erstrecken sich die Längsschlitze der Punktionskanüle über den Einführtrichter bis zu einer Anschlagplatte. Hierdurch ist das Aufspreizen der Zungen beim Einführen der Sonde bzw. ihrer Rückhalteplatte infolge der Abwinklung der im Bereich zwischen Einführtrichter und dem i. w. gerade verlaufenden Schaftabschnitt der Punktionskanüle nochmals verbessert.

Die Schiebehilfe selbst ist als Rohrstück ausgebildet und kann aus Metall und/oder Kunststoff bestehen.

Um eine kontrollierte Führung der Rückhalteplatte durch das Gewebe beim Setzen der Sonde bzw. des Katheters zu erreichen, ist es nach einer besonderen Ausgestaltung der Erfindung vorgesehen, daß in die Punktionskanüle ein Hülsenstück mit einem dem eingangsseitigen Durchmesser des Einführtrichters i. w. entsprechenden Außendurchmesser einschiebbar ist, in welche die Schiebehilfe mit Rückhalteplatte des Instrumentes einführbar ist. Eine Verletzung des Gewebes außerhalb der Punktionsstelle beim Einführen der Sonde bzw. Katheters ist damit ausgeschlossen.

Um das Einführen des Hülsenstückes in die Punktionskanüle bzw. den Einführtrichter zu erleichtern, ist nach der Erfindung eine in das Hülsenstück einschiebbare Einführhilfe vorgesehen, welche in eingeschobener Stellung eine aus dem vorderen Hülsenende vorstehende, vorzugsweise abgerundete kegelstumpfförmige Spitze aufweist.

Die Handhabung des erfindungsgemäßen Bestecks läßt sich noch dadurch verbessern, daß das Hülsenstück an seinem hinteren Ende eine radial nach außen weisende Anschlagplatte aufweist, gegen welche die Einführhilfe in eingeschobener Stellung in dem Hülsenstück mit ihrem Griffteil anliegt.

Die Punktionskanüle, das Hülsenstück und/oder die Einführhilfe können bspw. aus Kunststoff bestehen.

Herstellungstechnisch besonders günstig ist es auch, wenn die Punktionsnadel an einen die Führungen für die Zungen der Funktionskanüle aufweisenden, den Schaft der Punktionsnadel bildenden Kunststoffteil mit am hinteren Ende angeordnetem Griffstück gehalten ist.

Weitere Ziele, Vorteile, Merkmale und Anwendungsmöglichkeiten der vorliegenden Erfindung ergeben sich aus der nachfolgenden Beschreibung eines Ausführungsbeispieles anhand der Zeichnung.

Es zeigen:
- Figur 1: einen Halbschnitt in Längsrichtung durch eine mögliche Ausführungsform der erfindungsgemäßen Punktionskanüle mit eingesteckter Punktionsnadel,
- Figur 2: einen Querschnitt längs der Schnittlinie 2-2 gemäß Figur 1,
- Figur 3: einen Halbschnitt in Längsrichtung einer möglichen Ausführungsform eines erfindungsgemäßen Hülsenstückes mit eingesteckter Einführhilfe,
- Figur 4: einen Halbschnitt in Längsrichtung durch die Punktionskanüle mit eingestecktem Hülsenteil und Einführhilfe,
- Figur 5: einen Längsschnitt durch eine mögliche Ausführungsform einer erfindungsgemäßen Schiebehilfe mit Sonde,
- Figur 6: einen Längsschnitt durch die Punktionskanüle mit eingeschobener Schiebehilfe mit Sonde und
- Figur 7: eine fertig plazierte Sonde.

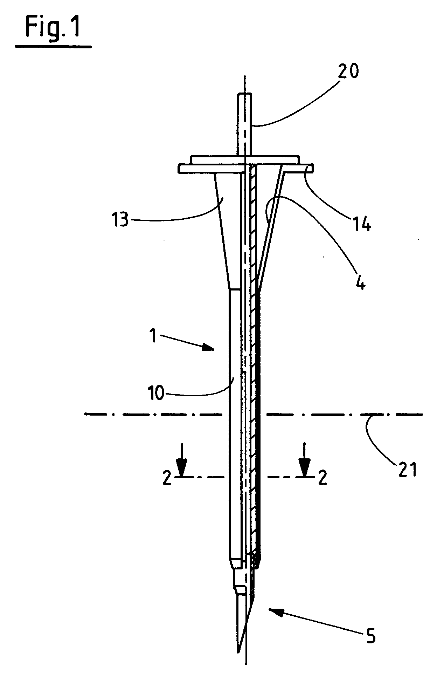

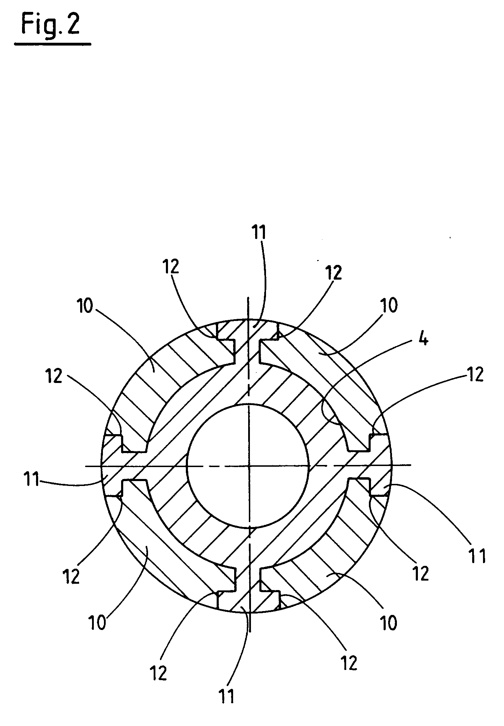

Das in den Figuren dargestellte Applikationsbesteck dient bei dem hier gewählten Ausführungsbeispiel zur perkutanen Gastrostomie. Selbstverständlich kann ein solches Applikationsbesteck auch für jede andere Art des Plazierens von Kathetern oder Sonden eingesetzt werden. Es weist gemäß Figur 1 eine aus Kunststoff bestehende Punktionskanüle 1 auf, in welche zum anfänglichen Punktieren eine Punktionsnadel 5 eingesteckt ist. Die Punktionskanüle 1 besitzt vier durchgehende Längsschlitze 9, wobei, wie aus Figur 2 zu ersehen, die durch die Längsschlitze 9 gebildeten kreissegmentförmigen Zungen 10 mittels umfangsseitig des Schaftes der Punktiernadel 5 vorgesehenen Führungen 11 zusammengehalten sind, um ein Einführen der Punktierkanüle 1 mit Punktionsnadel 5 ohne weitere Verletzung des Gewebes um die Punktierstelle zu ermöglichen.

Bei dem hier gewählten Ausführungsbeispiel sind die Führungen am Schaft der Punktiernadel 5 als nach radial außen vorstehende, i. w. T-förmig ausgebildete und in Längsrichtung der Punktiernadel 5 verlaufende Führungsstege 11 ausgeführt, in welche die kreissegmentförmigen Zungen 10 der Punktionskanüle 1 mit etwa stufenförmig abgesetzten Seitenrändern 12 eingreifen. In eingeschobener Stellung der Punktionsnadel 5 in die Punktionskanüle 1 ergänzen sich die Führungsstege 11 mit den dazwischen liegenden Zungen 10 zu einer i. w. geschlossenen zylindrischen Außenmantelfläche.

Die Punktionskanüle 1 weist an ihrem hinteren Ende einen Einführtrichter 13 mit endseitiger Anschlagplatte 14 auf, welche das Einführen der Punktionsnadel 5 und des weiter unten noch zu beschreibenden Hülsenstückes 15 erleichtern soll.

Nach dem Punktieren wird die Punktionsnadel 5 aus der Punktionskanüle 1 herausgezogen, wobei letztere im Körper verbleibt. Beim Herausziehen der Punktionsnadel 5 löst sich die Verbindung zwischen den Zungen 10 der Punktionskanüle 1.

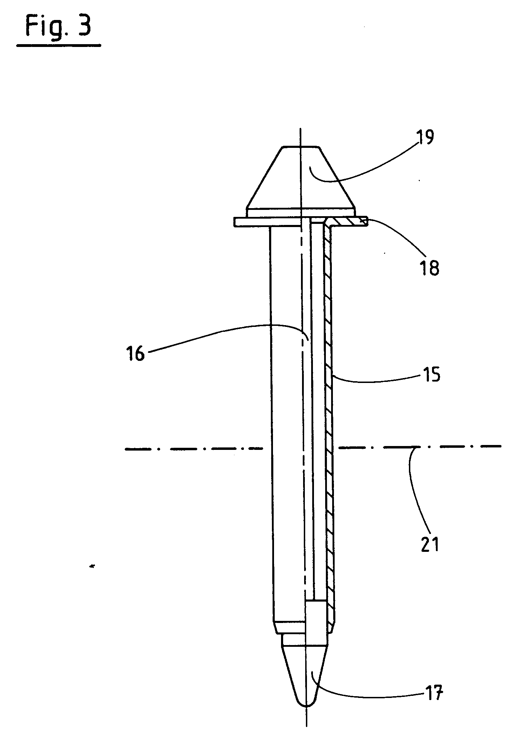

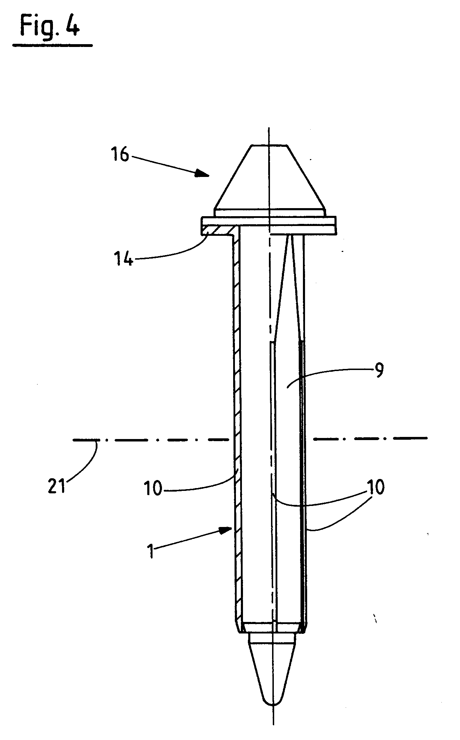

Sodann wird ein Hülsenstück 15 mit darin eingeschobener Einführhilfe 16 über den Einführtrichter 13 in die Punktionskanüle 1 eingeschoben, wie dies in Figur 4 dargestellt ist. Die Einführhilfe 16 weist gemäß Figur 3 in eingeschobener Stellung eine aus dem vorderen Hülsenende vorstehende, abgerundete kegelstumpfförmige Spitze 17 auf. Wie weiterhin aus Figur 3 zu ersehen, ist das Hülsenstück 15 an seinem hinteren Ende mit einer radial nach außen weisenden Anschlagplatte 18 versehen, gegen welche die Einführhilfe 16 in eingeschobener Stellung in dem Hülsenstück 15 mit ihrem Griffteil 19 anliegt.

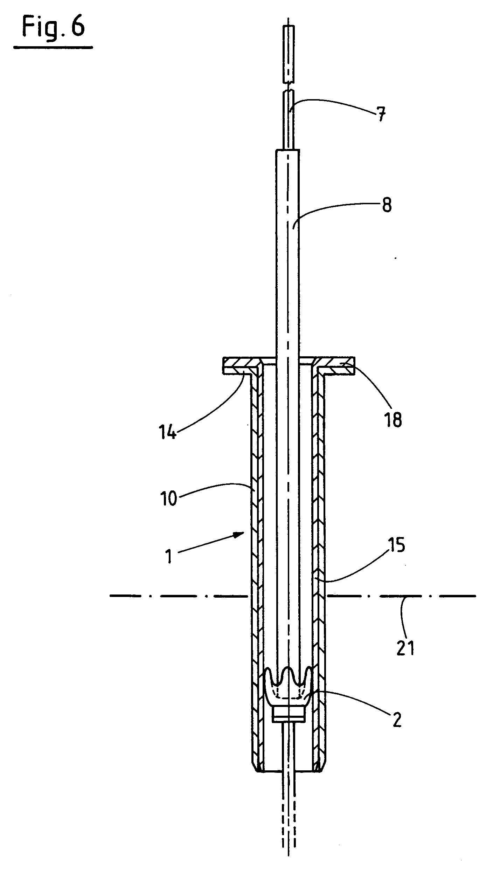

Gemäß Figur 6 werden beim Einschieben des Hülsenstückes 15 in die Punktionskanüle 1 deren Zungen 10 gespreizt, so daß das Hülsenstück 15 mit seinem vorderen Ende bis in den Magen gelangt. Das Aufspreizen der Zungen 10 wird dadurch unterstützt, daß beim Einschieben der Einführhilfe 16 aufgrund der Abwinklung der Zungen 10 im Übergangsbereich zwischen Einführtrichter 13 und gerade verlaufenden Zungenabschnitten im Schaftbereich der Punktionskanüle 1 die Zungen 10 nach radial außen gedrückt werden. Die Zungenabschnitte im Bereich des Einführtrichters 13 legen sich dabei an das Hülsenstück 15 an.

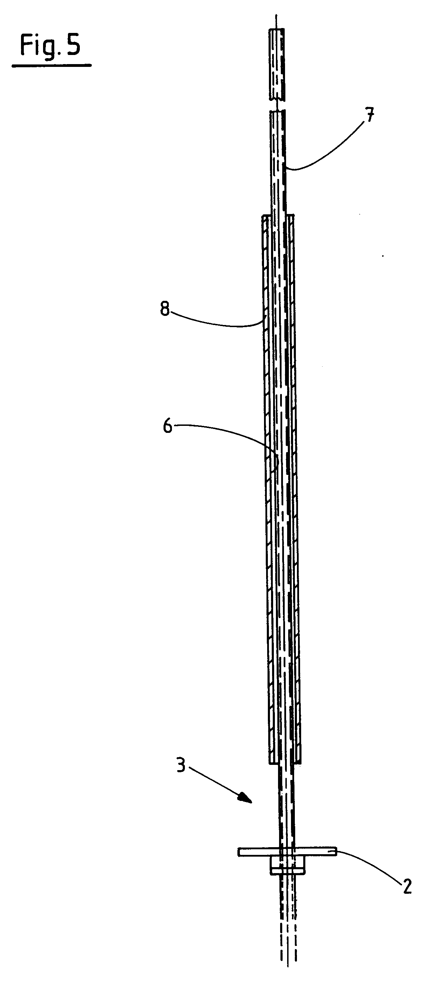

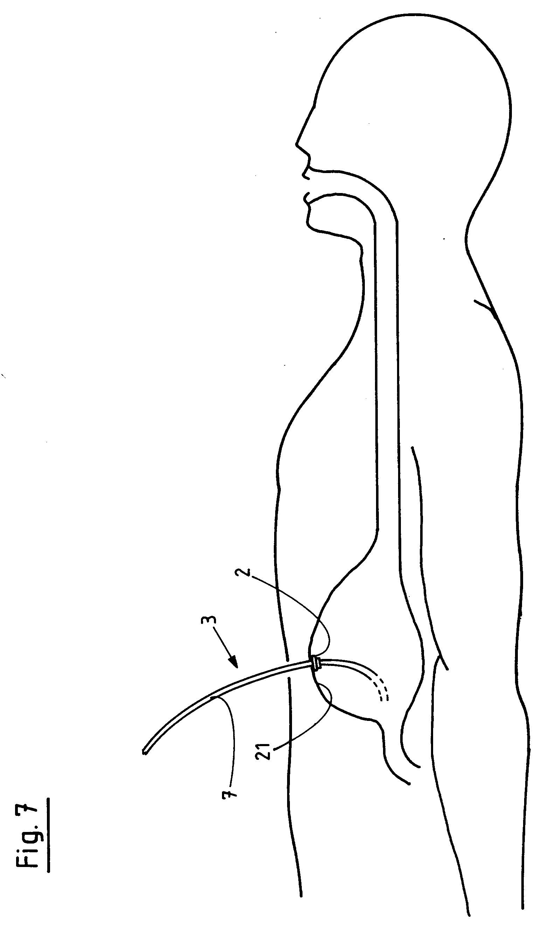

Sodann wird die Einführhilfe 16 herausgezogen und die Sonde 3 eingebracht. Hierzu wird, wie aus Figur 5 ersichtlich, die Sonde 3 mit ihrem Schlauch 7 durch den Durchgang 6 einer rohrförmige Schiebehilfe 8 hindurchgeführt, bis die Rückhalteplatte 2 der Sonde am vorderen Ende der Schiebehilfe 8 anliegt. Sodann wird die Schiebehilfe 8 mit der Rückhalteplatte 2 voran in das Hülsenstück 15 eingeführt, wie dies in Figur 6 gezeigt ist. Beim Einschieben legt sich die aus Silikonmaterial bestehende elastische Rückhalteplatte 2 entgegengesetzt der Schieberichtung nach hinten um, so daß ein Gleiten der Rückhalteplatte 2 innerhalb des Hülsenstückes 15 problemlos ermöglicht ist, ohne daß Gewebe im Bereich außerhalb der Punktionsstelle verletzt wird. Danach wird die Schiebehilfe 8 aus dem Hülsenstück 15 herausgezogen. Die Sonde 3 wird durch langsamen Zug am distalen Fadenende intragastral plaziert. Schließlich wird die Sonde 3 zusammen mit der Punktionskanüle 1 durch die Bauchwand nach außen gezogen, bis gemäß Figur 7 die Rückhalteplatte 2 an der Mageninnenwand 21 anliegt.

Die Punktionskanüle 1, das Hülsenstück 15 und/oder die Einführhilfe 16 bestehen bevorzugt aus Kunststoff. Die aus Metall bestehende Punktionsnadel 5 ist an einen die Führungsstelle 11 für die Zungen 10 der Punktionskanüle 1 aufweisenden Kunststoffteil mit am hinteren Ende angeordneten Griffstück 20 gehalten.

### Bezugszeichenliste

- 1: - Punktionskanüle
- 2: - Rückhalteplatte
- 3: - Sonde, Instrument
- 4: - Durchführung
- 5: - Punktionsnadel
- 6: - Durchgang
- 7: - Instrumentenschlauch
- 8: - Schiebehilfe
- 9: - Längsschlitze
- 10: - Zungen
- 11: - Führung, Steg
- 12: - Seitenrand
- 13: - Einführtrichter
- 14: - Anschlagplatte
- 15: - Hülsenstück
- 16: - Einführhilfe
- 17: - Spitze
- 18: - Anschlagplatte
- 19: - Griffteil
- 20: - Griffstück
- 21: - Mageninnenwand

## Patentansprüche

1. Applikationsbesteck in Kombination mit einem schlauch- oder röhrenförmigen Instrument (3) mit einem Instrumentenschlauch, wie Sonden, Katheter o. dgl., zur chirurgischen oder therapeutischen Behandlung des menschlichen oder tierischen Körpers oder zur Anwendung im Diagnostizierverfahren, wobei das Applikationsbesteck eine Punktionskanüle (1) mit einer Durchführung (4) mit wenigstens einem Längsschlitz (9) aufweist, **dadurch gekennzeichnet,** daß das Applikationsbesteck eine in die Punktionskanüle (1) verschieblich aufgenommene Punktionsnadel (5) aufweist, daß das Instrument (3) eine elastische Rückhalteplatte (2) aufweist, wobei das Instrument (3) nach Entfernen der Punktionsnadel (5) mit seiner Rückhalteplatte (2) voran mittels einer einen Durchgang (6) für den Instrumentenschlauch (7) aufweisenden Schiebehilfe (8) durch die Punktionskanüle (1) unter radialem Aufweiten ihrer längstgeschlitzten Durchführung (4) schiebbar und somit in den Körper einführbar ist.

2. Applikationsbesteck nach Anspruch 1, dadurch gekennzeichnet, daß die Punktionskanüle (1) im Bereich ihres Schaftes (4), vorzugsweise vier Längsschlitze mit am vorderen Ende der Punktionskanüle (1) angeordneten Sollbruchstellen aufweist, welche beim Durchschieben der Schiebehilfe (8) mit Rückhalteplatte (2) unter Aufspreizen der durch die Längsschlitze gebildeten segmentförmigen Zungen aufbrechen.

3. Applikationsbesteck nach Anspruch 1, dadurch gekennzeichnet, daß die Punktionskanüle (1) im Bereich ihres Schaftes (4), vorzugsweise vier Längsschlitze (9) aufweist, wobei durch die Längsschlitze (9) gebildeten segmentförmigen Zungen (10) mittels umfangsseitig der Punktiemadel (5) gebildeten Führungen (11) zusammengehalten und beim Einführen der Schiebehilfe (8) mit der Rückhalteplatte (2) aufspreizbar sind.

4. Applikationsbesteck nach Anspruch 3, dadurch gekennzeichnet, daß die Führungen an der Punktiernadel (5) als nach radial außen vorstehende, in Längsrichtung der Punktionsnadel (5) verlaufende Führungsstege (11) ausgebildet sind, welche sich mit den dazwischen zu liegen kommenden Zungen (10) zu einer i. w. geschlossenen zylindrischen Außenmantelfläche ergänzen.

5. Applikationsbesteck nach Anspruch 3 oder 4, dadurch gekennzeichnet, daß die Führungsstege (11) i. w. T-förmig ausgebildet und von den Zungen (10) mit etwa stufenförmig abgesetzten Seitenrändern (12) hintergreifbar sind.

6. Applikationsbesteck nach einem der Ansprüche 1 bis 5, dadurch gekennzeichnet, daß die Punktionskanüle (1) an ihrem hinteren Ende einen Einführtrichter (13) aufweist.

7. Applikationsbesteck nach einem der Ansprüche 2 bis 5, dadurch gekennzeichnet, daß sich die Längsschlitze (9) der Punktionskanüle (1) über den Einführtrichter (13) bis zu einer am hinteren Ende der Punktionskanüle (1) angeordneten Anschlagplatte (14) erstrecken.

8. Applikationsbesteck nach einem der Ansprüche 1 bis 7, dadurch gekennzeichnet, daß die Schiebehilfe (8) als Rohrstück ausgebildet.

9. Applikationsbesteck nach einem der Ansprüche 1 bis 8, dadurch gekennzeichnet, daß in die Punktionskanüle (1) ein Hülsenstück (15) mit einem dem eingangsseitigen Durchmesser des Einführtrichters (13) i. w. entsprechenden Außendurchmesser einschiebbar ist, in welches die Schiebehilfe (8) mit Rückhalteplatte (2) des Instrumentes (3) einführbar ist.

10. Applikationsbesteck nach Anspruch 9, dadurch gekennzeichnet, daß zum Einführen des Hülsenstückes (15) die Punktionskanüle (1) eine in das Hülsenstück (15) einschiebbare Einführhilfe (16) vorgesehen ist, welche in eingeschobener Stellung eine aus dem vorderen Hülsenende vorstehende, vorzugsweise abgerundete kegelstumpfförmige Spitze (17) aufweist.

11. Applikationsbesteck nach Anspruch 9 oder 10, dadurch gekennzeichnet, daß das Hülsenstück (15) an seinem hinteren Ende eine radial nach außen weisende Anschlagplatte (18) aufweist, gegen welche die Einführhilfe (16) in eingeschobener Stellung in dem Hülsenstück (15) mit ihrem Griffteil (19) anliegt.

12. Applikationsbesteck nach einem der vorhergehenden Ansprüche, dadurch gekennzeichnet, daß die Punktionskanüle (1), das Hülsenstück (15), die Einführhilfe (16) und/oder die Schiebehilfe (8) aus Kunststoff bestehen.

13. Applikationsbesteck nach einem der vorhergehenden Ansprüche, dadurch gekennzeichnet, daß die Punktionsnadel (5) an einen die Führungen (11) für die Zungen (10) der Punktionskanüle (1) aufweisenden Kunststoffteil mit am hinteren Ende angeordnetem Griffstück (20) gehalten ist.

## Claims

1. Application instruments set with a hose-shaped or tubular instrument (3) with an instrument hose, such as probes, catheters or the like for the surgical or therapeutic treatment of the human or animal body or for application in the diagnostic process, wherein the application instruments set comprises an aspiration cannula (1) with a bushing (4) with at least one longitudinal slot (9), characterised thereby, that the application instruments set comprises an aspiration needle (5) received in the aspiration cannula (1) to be displaceable and that the instrument (3) comprises an elastic retaining plate (2), wherein the instrument (3) after removal of the aspiration needle (5) with its retaining plate (2) is pushable forward through the aspiration cannula (1), whilst radially enlarging its longitudinal bushing (4), by means of a pushing aid (8) displaying a passage (6) for the instrument hose (7) and is thus introducible into the body.

2. Application instruments set according to claim 1, characterised thereby, that the aspiration cannula (1) in the region of its shank (4) displays preferably four longitudinal slots with frangible places which are arranged at the forward end of the aspiration cannula (1) and which during the pushing-through of the pushing aid (8) with retaining plate (2) break open whilst splaying out the segment-shaped tongues formed by the longitudinal slots.

3. Application instruments set according to claim 1, characterised thereby, that the aspiration cannula (1) in the region of its shank (4) displays preferably four longitudinal slots (9), wherein segment-shaped tongues (10) formed by the longitudinal slots (9) are held together by means of guides (11) formed circumferentially of the aspiration needle (5) and are splayable open during the introduction of the pushing aid (8) with retaining plate (2).

4. Application instruments set according to claim 3, characterised thereby, that the guides at the aspiration needle (5) are formed as guide webs (11), which extend in longitudinal direction of the aspiration needle (5), protrude radially outwards and which together with the tongues (10) coming to lie therebetween make up a substantially closed cylindrical outer casing surface.

5. Application instruments set according to claim 3 or 4, characterised thereby, that the guide webs (11) are formed to be substantially T-shaped and engageable behind by the tongues (10) by side rims (12) set back in about step shape.

6. Application instruments set according to one of the claims 1 to 5, characterised thereby, that the aspiration cannula (1) comprises an entry funnel (13) at its rear end.

7. Application instruments set according to one of the claims 2 to 5, characterised thereby, that the longitudinal slots (9) of the aspiration cannula (1) extend beyond the entry funnel (13) to an abutment plate (14) arranged at the rear end of the aspiration cannula (1).

8. Application instruments set according to one of the claims 1 to 7, characterised thereby, that the pushing aid (8) is formed as tubular member.

9. Application instruments set according to one of the claims 1 to 8, characterised thereby, that a sleeve member (15), into which the pushing aid (8) with retaining plate (2) of the instrument (3) is introducible, with an external diameter substantially corresponding with the entry diameter of the entry funnel (13), is pushable into the aspiration cannula (1).

10. Application instruments set according to claim 9, characterised thereby, that for the introduction of the sleeve member (15), the aspiration cannula (1) is provided with an entry aid (16), which is pushable into the sleeve member (15) and in the pushed-in setting displays a frusto-conical tip (17), which protrudes from the forward sleeve end and is preferably rounded off.

11. Application instruments set according to claim 9 or 10, characterised thereby, that the sleeve member (15) at its rear end displays an abutment plate (18), which faces radially outwards and against which the entry aid (16) in the pushed-in setting in the sleeve member (15) lies by its handle part (19).

12. Application instruments set according to one of the preceding claims, characterised thereby, that the aspiration cannula (1), the sleeve member (15), the entry aid (16) and/or the pushing aid (8) consist of synthetic material.

13. Application instruments set according to one of the preceding claims, characterised thereby, that the aspiration needle (5) is retained at a synthetic material part, which comprises the guides (11) for the tongues (10) of the aspiration cannula (1), with a handle member (20) arranged at the rear end.

## Revendications

1. Equipement applicateur en combinaison avec un instrument (3) en forme de tuyau souple ou de tube comportant un tuyau souple d'instrument, tel que sonde, cathéter ou analogue, servant à un traitement chirurgical ou thérapeutique du corps humain ou animal ou à une application dans un procédé de diagnostic, équipement applicateur comprenant une canule de ponction (1) comportant un passage (4) présentant au moins une fente longitudinale (9), caractérisé en ce que l'équipement applicateur comprend une aiguille de ponction (5) logée d'une manière coulissante dans la canule de ponction (1) et en ce que l'instrument (3) comprend une plaquette élastique de retenue (2), de sorte qu'une fois l'aiguille de ponction (5) retirée, on peut faire coulisser l'instrument (3), avec sa plaquette de retenue (2) dirigée vers l'avant, à travers la canule de ponction (1) moyennant un élargissement radial de son passage (4) fendu longitudinalement, au moyen d'un auxiliaire de coulissement (8) comportant un passage (6) pour le tuyau souple d'instrument (7), et qu'on peut ainsi introduire l'instrument (3) dans le corps.

2. Equipement applicateur suivant la revendication 1, caractérisé en ce que la canule de ponction (1) présente, dans la zone de sa tige (4), de préférence quatre fentes longitudinales comportant des emplacements de rupture obligée, disposés à l'extrémité avant de la canule de ponction (1), qui se rompent lors de la traversée coulissante de l'auxiliaire de coulissement (8) avec la plaquette de retenue (2), avec un déploiement des languettes en forme de segment formées par les fentes longitudinales.

3. Equipement applicateur suivant la revendication 1, caractérisé en ce que, dans la zone de sa tige (4), la canule de ponction (1) comporte de préférence quatre fentes longitudinales (9), les languettes en forme de segments (10) formées par les fentes longitudinales (9) étant maintenues assemblées au moyen de glissières (11), formées sur la face périphérique de l'aiguille de ponction (5), et pouvant être déployées lors de l'introduction de l'auxiliaire de coulissement (8) avec la plaquette de retenue (2).

4. Equipement applicateur suivant la revendication 3, caractérisé en ce que les glissières situées sur l'aiguille de ponction (5) sont réalisées sous forme de nervures de guidage (11) qui font saillie radialement vers l'extérieur et s'étendent suivant la direction longitudinale de l'aiguille de ponction (5) et qui complètent les languettes (10), venant se placer entre elles, pour former une surface périphérique extérieure cylindrique essentiellement fermée.

5. Equipement applicateur suivant la revendication 3 ou 4, caractérisé en ce que les nervures de guidage (11) sont réalisées essentiellement en forme de T et les languettes (10) peuvent venir s'accrocher, par des bords latéraux (12) à épaulement créant une forme étagés, derrière ces nervures de guidage (11).

6. Equipement applicateur suivant l'une des revendications 1 à 5, caractérisé en ce que la canule de ponction (1) comporte un entonnoir d'entrée (13) à son extrémité arrière.

7. Equipement applicateur suivant l'une des revendications 2 à 5, caractérisé en ce que les fentes longitudinales (9) de la canule de ponction (1) s'étendent sur l'entonnoir d'entrée (13) jusqu'à une plaquette de butée (14) disposée à l'extrémité arrière de la canule de ponction (1).

8. Equipement applicateur suivant l'une des revendications 1 à 7, caractérisé en ce que l'auxiliaire de coulissement (8) est réalisé sous forme d'une pièce tubulaire.

9. Equipement applicateur suivant l'une des revendications 1 à 8, caractérisé en ce que, dans la canule de ponction (1), il est possible d'introduire de manière coulissante une pièce formant manchon (15) qui présente un diamètre extérieur correspondant essentiellement au diamètre côté d'entrée de l'entonnoir d'entrée (13) et dans laquelle l'auxiliaire de coulissement (8), avec la plaquette de retenue (2) de l'instrument (3), peut être introduit.

10. Equipement applicateur suivant la revendication 9, caractérisé en ce que, pour l'introduction de la pièce formant manchon (15) dans la canule de ponction (1), il est prévu un auxiliaire d'introduction (16) qui peut être introduit d'une manière coulissante dans la pièce formant manchon (15) et qui, dans la position enfoncée, présente une pointe (17), de préférence de forme tronconique arrondie, qui fait saillie hors de l'extrémité avant du manchon.

11. Equipement applicateur suivant la revendication 9 ou 10, caractérisé en ce que la pièce formant manchon (15) comporte, à son extrémité arrière, une plaquette de butée (18) dirigée radialement vers l'extérieur sur laquelle l'auxiliaire d'introduction (16) prend appui par sa partie de préhension (19) dans la position enfoncée dans la pièce formant manchon (15).

12. Equipement applicateur suivant l'une des revendications précédentes, caractérisé en ce que la canule de ponction (1), la pièce formant manchon (15), l'auxiliaire d'introduction (16) et/ou l'auxiliaire de coulissement (8) sont en matière plastique.

13. Equipement applicateur suivant l'une des revendications précédentes, caractérisé en ce que l'aiguille de ponction (5) est maintenue, par une partie de préhension (20) disposée à l'extrémité arrière, sur une pièce en matière plastique comportant les glissières (11) prévues pour les languettes (10) de la canule de ponction (1).
